# EUROPÄISCHE PATENTSCHRIFT

(11) **EP 0 789 576 B1**
(45) Veröffentlichungstag und Bekanntmachung des Hinweises auf die Patenterteilung: **10.07.2002**
(21) Anmeldenummer: 95940110.0
(22) Anmeldetag: 06.11.1995
(51) Int. Cl.: A61K 35/78, A61P 37/08

(54) **EXTRAKT VON CRATAEGUS UND SEINE VERWENDUNG**
EXTRACT OF CRATAEGUS AND ITS USE
EXTRAIT DE CRATAEGUS ET SON UTILISATION

(30) Priorität: 05.11.1994 DE 4439613
(43) Veröffentlichungstag der Anmeldung: 20.08.1997
(73) Patentinhaber: SCHAPER & BRÜMMER GMBH & CO. KG, D-38259 Salzgitter (DE)
(72) Erfinder: SCHNEIDER, Peter, D-38259 Salzgitter (DE); BEUSCHER, Norbert, D-38259 Salzgitter (DE)
(74) Vertreter: Lins, Edgar, Dipl.-Phys. Dr.jur.
(86) Internationale Anmeldenummer: DE9501531
(87) Internationale Veröffentlichungsnummer: WO9614078

(56) Entgegenhaltungen:
- DE-B- 1 101 697
- DE-C- 876 441
- DE-C- 908 908
- ARZNEIMITTEL FORSCHUNG DRUG RESEARCH., Bd. 17, Nr. 4, 1967, AULENDORF DE, Seiten 490-491, XP002000953

## Beschreibung

Die Erfindung betrifft einen Extrakt von Blättern, Blüten und/oder Früchten von Crataegus monogyna oder Crataegus oxyacantha. Die Erfindung betrifft ferner eine Verwendung dieses Extraktes zur Herstellung eines Arzneimittels.

Gemäß der DE-A-876 441 beinhaltet ein Weißdornextrakt, der beispielsweise mit Äther hergestellt ist, ein Produkt mit einem Schmelzpunkt zwischen 262 und 264°, das aus zwei Bestandteilen besteht, die sich voneinander trennen lassen. Die Trennung kann mit Alkohol oder durch Wasserentzug beim Erhitzen vorgenommen werden. Mit der Droge können Herzerkrankungen behandelt werden, wobei eine gefäßverengende oder auch eine gefäßerweiternde Wirkung erzielbar ist.

In DE-A-1 101 697 ist beschrieben, dass Crataegus wasserlösliche und wasserunlösliche Inhaltsstoffe enthält, die mit verschiedenen Extraktionsmitteln gewonnen werden können. Erwähnt ist eine medizinische Wirkung bei Herzerkrankungen. Es sind als Crataegusextrakte hergestellte Arzneimittel im Markt, die standardisiert sind, beispielsweise auf eine vorgegebene Konzentration von poligomeren Procyanidinen oder auf eine vorgegebene Konzentration von kondensierten Flavonen (Esbericard®, s. Heilpflanzen-Lexikon für Ärzte und Apotheker, Stuttgart/New York 1987, Seiten 82/83). Sie sind als Herzmittel bezeichnet. In Fischer/Krug "Heilkräuter und Arzneipflanzen", Heidelberg 1984 ist die pharmazeutische Wirkung von Crataegus auch für Asthma cardiale angegeben. Asthma cardiale bezeichnet Atemnotzustände, die durch Lungenödeme als Folge einer Linksherzinsuffizienz verursacht werden.

Überraschender Weise hat ein erfindungsgemäß modifizierten Crataegusextrakt Wirksamkeiten gegen altergische Erkrankungen und gegen intrinsisches oder extrinsisches Asthma bronchiale.

Allergische Erkrankungen vom Soforttyp (Typ I-Allergie) sind zum großen Teil durch eine erhöhte Immunglobulin E (IgE)-Synthese, u. a. verursacht durch Allergene aus der Umwelt, charakterisiert. Als Beispiel für eine allergische Erkrankung vom Soforttyp ist die allergische Rhinitis (Heuschnupfen) des Menschen zu nennen.

Neben der Allergie vom Typ I gibt es weitere Allergieformen (zytotoxische und immunkomplexvermittelte Formen (Typen II und III) und die Spätform (Typ IV-Allergie)). Typische Erkrankungen vom Typ IV sind Kontaktekzeme, die chronische extrinsische allergische Alveolitis und die iatrogene, interstitielle Pneumopathie.

Die Erkennung des Allergens durch das Immunsystem erfolgt durch Major-Histocompatibility-Complex-Class II-positive Zellen und spezifische T-Helfer-Lymphozyten. Die T-Zellen stimulieren B-Zellen zur Produktion von Allergen-spezifischem IgE. Das sezernierte IgE bindet bevorzugt an Fc-epsilon-Rezeptoren auf Mastzellen und Basophilen, wodurch diese sensibilisiert werden. Die Rezeptoren sind über einen zweiten Ast mit dem Allergen verbunden ("Bridging").

In der Folge des Bridgings werden Histamin, Leukotriene und andere sogenannte Mediatoren durch die Zelle ausgeschüttet. Als wichtigste Mediatoren gelten Histamin, Bradykinin, Prostaglandine, Prostacycline, Leukotriene und PAF (Plättchen-aktivierender Faktor).

Die Mediatorenausschüttung kann Entzündungen zur Folge haben.

Das bronchiale Asthma ist die häufigste chronische Erkrankung bei Kindern. In der Pathogenese des Asthmas ist die Bedeutung der Entzündung der Atemwege, bestehend aus einer ödematösen und einer zellulären Komponente, seit langem bekannt.

Selbst bei milden Formen des Asthmas wurde eine Entzündung der Atemwege nachgewiesen. An dem Entzündungsprozeß sind residente Gewebszellen beteiligt, wie Epithelzellen, Mastzellen und Fibroblasten. Diese Zellen produzieren die genannten Entzündungsmediatoren. Der Auslöser der asthmatischen Entzündungsreaktion ist gewöhnlich ein Allergen; jedoch können auch nichtallergene Trigger, wie Viren und Irritantien, die Entzündung induzieren. Die Produkte pulmonärer Mastzellen initiieren eine Bronchokonstriktion, wobei andere Zellen, wie Makrophagen und Lymphozyten, die IgE-Rezeptoren besitzen, ebenfalls beteiligt sind. Andere Entzündungszellen werden von den von Mastzellen ausgeschleusten Mediatoren in den Respirationstrakt gelockt. Sind die weiteren Entzündungszellen dort angelangt, weitet sich die Entzündung aus. Verschiedene Faktoren, wie Zigarettenrauch, Viren, Reizstoffe oder Allergene können zur Exazerbation des Asthmas führen.

Allergien werden bisher zumeist prophylaktisch mit Cromoglicinsäure und ihren Derivaten, bzw. therapeutisch mit Antihistaminika behandelt, die die Histaminrezeptoren blockieren. Für die Behandlung asthmatischer Erkrankungen kommen darüber hinaus Corticosteroide, β₂-Agonisten, Xanthinderivate und nichtsteroidale, antientzündliche Substanzen zur Anwendung.

Während die Prophylaxe mit Cromoglicinsäure oft an mangelhafter Compliance der Patienten, infolge der unbequemen Handhabung der Präparate scheitert, ist die Therapie mit den übrigen Substanzen mit zum Teil starken Nebenwirkungen behaftet. Außerdem wurde für Cromoglicinsäure und Nedocromil-Natrium eine starke Tachyphylaxie (Wirkungsminderung nach mehrfacher Applikation in kurzen Zeitabständen) nachgewiesen. Es besteht daher Bedarf an einer risikoarmen Behandlungsmethode, die keinen der zuvor erwähnten Nachteile hat. Eine Therapie mit einem nebenwirkungsfreien Phythotherapeutikum wäre vor allem für Kinder die Therapie der Wahl.

Verschiedene Präparate aus Crataegus monogyna (auch Crataegus laevigata), Crataegus oxyacantha oder anderen im gültigen Deutschen Arzeibuch aufgeführten Crataegus-Arten (Weißdornblätter mit Blüten) werden bisher nach der Monographie der Kommission E beim Bundesgesundheitsamt (Bundesanzeiger Jahrgang 46, Nr. 133 vom 19. Juli 1994, S. 7360) bei nachlassender Leistungsfähigkeit des Herzens entsprechend Stadium II nach NYHA angewandt. Früchte des Weißdorn unterscheiden sich in den pharmakologischen Eigenschaften, sowie der Pharmakokinetik und Toxikologie nur wenig von der Droge Weißdornblätter mit Blüten. Als Wirkstoffe für diese Indikationen wurden bisher Flavonoide, Flavane und Procyanidine nachgewiesen.
Eine andere Verwendung ist nicht bekannt.

Kondensierbare Flavane sind 3-Deoxycatechinderivate; sie werden aus dem Spezialextrakt durch Erhitzung unter Zugabe von Salzsäure ausgefällt und können nach Trocknung gravimetrisch bestimmt werden.

Bezüglich des Wirkungsmechanismus von Crataeguspräparationen und -inhaltsstoffen wurde eine Hemmung der Aktivität der Phosphodiesterasen nachgewiesen.

Weiterhin wird durch Inhaltsstoffe aus Crataegus die Kapillarpermeabilität vermindert. Die Eigenschaft von Procyanidinen als Radikalfänger wurde beschrieben. Nach Untersuchungen der elektrophysiologischen Änderungen an der glatten Muskulatur menschlicher Koronararterien und am Papillarmuskel von Hunden unter der Einwirkung von Crataegus-Extrakt, die mechanisch zu einer Vasodilatation und zu einer Zunahme der Kontraktionsamplitude am Herzen führen, wurde gefunden , daß Crataegus-Extrakt als phytopharmakologischer K⁺-Kanalöffner bezeichnet werden kann.

Überraschend wurde nun gefunden, daß die pharmazeutische Wirksamkeit eines herkömmlichen Crataegus-Extraktes (Weißdornextrakt) für bestimmte Indikationen dadurch wesentlich erhöht werden kann, daß der Extrakt durch eine thermische Nachbehandlung modifiziert wird. Die thermische Nachbehandlung führt zur Bildung höhermolekularer kondensierbarer Flavane und läßt einige Bestandteile des Extraktes ausfallen. Durch Abfiltrieren der ausgefällten Bestandteile entsteht somit ein Spezialextrakt, dessen Anteil an kondensierbaren Flavanen höher ist als bei herkömmlichen Extrakten und der weitgehend von zytotoxischen Substanzen befreit ist.

Der erfindungsgemäße Spezialextrakt hemmt die Freisetzung des die Typ I-Allergie hervorrufenden IgE aus humanen Myelomzellen stark um bis zu ca. 80 %. Dieser Extrakt weist in einem Testsystem im untersuchten Konzentrationsbereich (50 - 500 µg/ml kondensierbarer Flavane) keine Zytotoxizität auf, so daß er auch in vivo bei Konzentrationen zwischen 5 und 100 ml keine Zytotoxizität aufweist.

Die durch ein auf die Haut aufgetragenes Allergen verursachte Lymphknotenproliferation bei Mäusen konnte durch intravenöse Verabreichung des Spezialextraktes stark vermindert werden.

Durch die Verabreichung des Spezialextraktes per Inhalation konnte der durch Acetylcholin ausgelöste Bronchospasmus stark gehemmt werden.

Die Herstellung des Spezialextraktes weist als ersten Verfahrensschritt die übliche Extraktion mit einem bekannten Extraktionsmittel auf. Beispielsweise wird die zerkleinerte Droge (Früchte und Blätter mit Blüten im Verhältnis 3:1) mit Aceton 40 % extrahiert. Die erfindungsgemäße Nachbehandlung des Extraktes sieht die Befreiung des Extraktes von Aceton und Lösen in gereinigtem Wasser vor. Der Extrakt wird einer Temperaturbehandlung unterworfen und filtriert. Vorzugsweise wird der Extrakt wiederholt auf eine Temperatur über 100 °C erhitzt und jeweils anschließend filtriert. In einem Ausführungsbeispiel ist diese Erhitzung und anschließende Filtration dreimal vorgenommen worden. Anschließend wird der Extrakt auf den gewünschten Gehalt an kondensierbaren Flavanen eingestellt.

Durch diese Vorgehensweise wird der Ausgangsextrakt von unerwünschten Eigenschaften befreit. Er ist unter den gewählten experimentellen Bedingungen nicht toxisch.

Neben der Verwendung des Spezialextraktes für die Behandlung allergischer Erkrankungen eignet sich der Spezialextrakt überraschend auch für die Behandlung von Asthmaerkrankungen aller Art, also auch nicht allergischer Asthmaerkrankungen.

Der Gehalt des Spezialextraktes an kondensierbaren Flavanen liegt zwischen 5 und 100 mg/ml.

Der Spezialextrakt zeigte in biologischen Untersuchungen folgenden Wirkungen:

### Beispiel 1:

### Untersuchung zur Hemmung der IgE-Freisetzung aus humanen U266-Zellen durch einen Spezialextrakt von Crataegus:

Die Wirkung der Inhaltsstoffe des Extraktes von Crataegus wurde in-vitro an Zellen der humanen Myelomlinie U266 (ATCC U266B1 IgE-Myelomzellen) geprüft, die spontan IgE lambda sezernieren.

Gleichzeitig wurde die Toxizität der Probe unter Verwendung eines fluorochromen Farbstoffes untersucht.

Unter Berücksichtigung der Ergebnisse des Toxizitätstestes ließ sich in dem Testsystem die Wirkung des Spezialextraktes auf die IgE-Sekretion bestimmen.

Die U266-Zellen wurden über drei Tage vermehrt (Medium: 85 % RPMI 1640 + 15 % FKS, Inkubation: bei 37 °C, 90 % rel. Luftfeuchte und 5 % CO₂). Nach 24 Stunden wurde die Testlösung mit den Konzentrationen 450, 225, 112.5 und 56.25 ug/ml an kondensierbaren Flavanen zugegeben (Doppelansatz). Nach weiteren 72 Stunden Inkubation erfolgte eine mikroskopische Beurteilung der Zellen, Zentrifugation und die Abnahme des Zellüberstandes. Der Gehalt an Gesamt-IgE wurde mit Hilfe eines quantitativen fluorometrischen Meßverfahrens bestimmt. Für die Versuchsansätze wurde die prozentuale Zu- oder Abnahme des IgE-Gehaltes im Vergleich zur Negativkontrolle ermittelt.

Nach 72 Stunden Inkubation wurde zu einem Parallelansatz der Zellen das Substrat 4-Methylumbelliferyl-heptanoat (4-MeUH) gegeben und der Ansatz für 2 Stunden bei Raumtemperatur inkubiert. Die anschließende fluoreszenzoptische Messung der Menge des entstandenen Methylumbelliferon gab Aufschluß über die Zytotoxizität der eingesetzten Probe gegenüber einer Negativkontrolle. Für die Versuchsansätze wurde die prozentuale Zuoder Abnahme der Extinktion im Vergleich zur Negativkontrolle ermittelt.

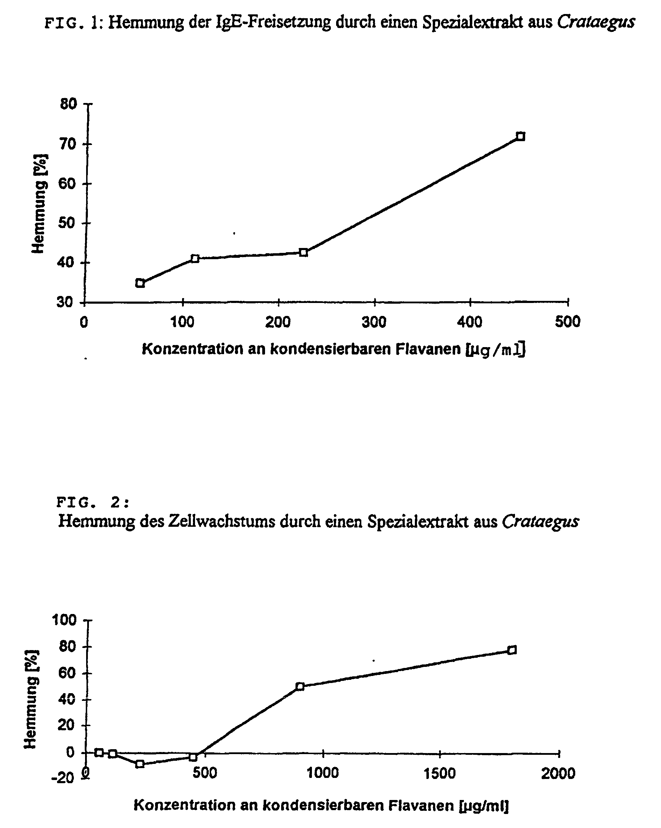

Die Ergebnisse der Untersuchung sind in Figur 1 zusammengefaßt. Daraus ergibt sich, daß die Freisetzung von Immunglobulin E aus humanen Myelomzellen durch Inhaltsstoffe des Extraktes von Crataegus bei einer zugesetzten Lösung mit 450 µg/ml kondensierbaren Flavanen im Testansatz um ca. 80 % gehemmt werden kann. Bei dieser Konzentration war für die Zellen keine toxische Wirkung nachweisbar (Figur 2).

### Beispiel 2:

### Untersuchung zur Hemmung der allergischen Lymphknotenproliferation durch einen Spezialextrakt von Crataegus:

Die Hemmung der durch ein Allergen verursachten Lymphknotenproliferation wurde in-vivo geprüft. Jungen, erwachsenen, weiblichen NMRI-Mäusen wurde an vier Tagen im Abstand von jeweils zwei Tagen ein starkes Allergen, gelöst in Aceton/Olivenöl 4:1, in die rasierten Kniefalten beider Seiten eingerieben. Mäuse der Kontrollgruppe erhielten auf gleiche Weise das Vehikel.

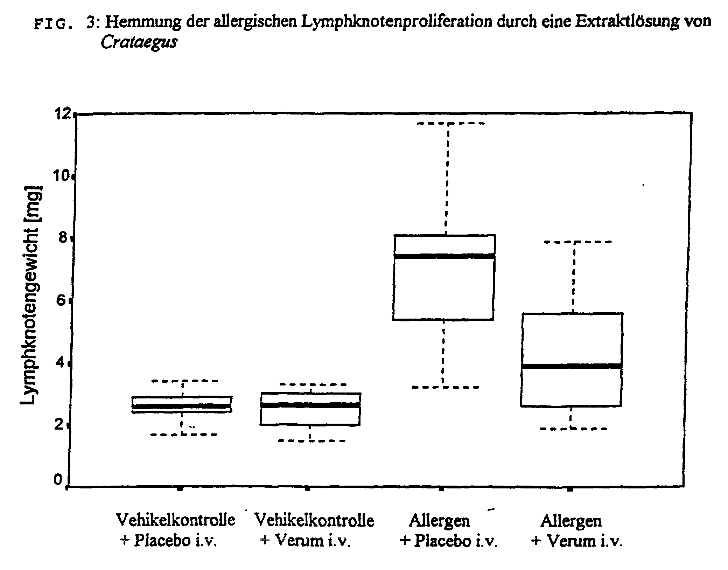

Jeweils zwei Stunden vor der Einreibung wurde den Tieren die Testsubstanz in wäßriger Lösung intravenös verabreicht. Zwei Tage nach der letzten Applikation wurden die Tiere getötet, und die Kniefaltenlymphknoten beider Seiten wurden präpariert und gewogen. Pro Tier wurde das durchschnittliche Lymphknotengewicht ermittelt, und die Unterschiede in den Lymphknotengewichten wurden zwischen Verumgruppe und Kontrollen bestimmt. Eine starke dosisabhängige Hemmung der Lymphknotenproliferation durch Inhaltsstoffe von Crataegus zeigt Figur 3.

Die Erfindung umfaßt die Verwendung eines Spezialextraktes aus Crataegus zur Behandlung allergischer und insbesondere asthmatischer Erkrankungen, die u.a. durch einen erhöhten Immunglobulin E-Gehalt des Blutserums und durch die Freisetzung von Mediatoren, charakterisiert sind. Klinische Beispiele für allergische Erkrankungen sind: Anaphylaxie, allergische Rhinitis, allergisches Asthma bronchiale, allergische Konjunktivitis, allergische Urticaria und allergische Gastroenteritis. Der bezeichnete Spezialextrakt ist zur Behandlung von allergischen Erkrankungen sämtlicher Organe geeignet. Der Extrakt kann enteral, parenteral, topisch oder durch Inhalation verabreicht werden.

Die Erfindung hat erhebliche klinische Bedeutung, da insbesondere bei Kindern Allergien und asthmatische Erkrankungen stark im Zunehmen begriffen sind und ein Mangel an nebenwirkungsarmen Therapeutika besteht.

## Patentansprüche

1. Extrakt von Blättern, Blüten und/oder Früchten von Crataegus monogyna oder Crataegus oxyacantha, herstellbar mit einem Verfahren mit folgenden Verfahrensschritten:
- Durchführung der Extraktion mit Aceton 40%
- Entfernung des Acetons und Herstellung einer wässrigen Lösung des Extraktes
- Erhitzen der wässrigen Lösung zur Bildung höhermolekularer kondensierbarer Flavane auf über 100° C
- Abfiltrieren der beim Erhitzen ausgefällten Bestandteile
- Wiederholung der Verfahrensschritte Erhitzen und Abfiltrieren und Einstellung eines Gehaltes an kondensierbaren Flavanen zwischen 5 und 100 mg/ml in der erhaltenen Lösung.

2. Verwendung des Extraktes nach Anspruch 1 zur Herstellung eines Arzneimittels zur Behandlung allergischer Erkrankungen.

3. Verwendung des Extraktes nach Anspruch 1 zur Herstellung eines Arzneimittels zur Behandlung von intrinsischem oder extrinsischem Asthma.

## Claims

1. An extract of leaves, flowers and/or fruits of *Crataegus monogyna* or *Crataegus oxyacantha*, produced by a process involving the following process steps:
- extraction using 40%-strength acetone
- removal of the acetone and preparation of an aqueous solution of the extract
- heating of the aqueous solution to more than 100 °C to form higher molecular condensable flavans
- removal, by filtration, of the components precipitated on heating
- repetition of the heating and filtering steps and adjustment of the content of condensable flavans in the resulting solution to a value between 5 and 100 mg/mL.

2. A method of using the extract as defined in claim 1 for the production of a pharamceutical for the treatment of allergic disorders.

3. A method of using the extract as defined in claim 1 for the production of a pharmaceutical for the treatment of intrinsic or extrinsic asthma.

## Revendications

1. Extrait de feuilles, de fleurs et/ou de fruits de Crataegus monogyna ou Crataegus oxyacantha, pouvant être fabriqué par un procédé comportant les étapes de procédé suivantes:
- effectuer l'extraction avec de l'acétone à 40%;
- enlever l'acétone et produire une solution aqueuse de l'extrait;
- chauffer la solution aqueuse à plus de 100°C pour former des flavanes condensables à poids moléculaire plus élevé;
- filtrer les composants précipités lors du chauffage;
- répéter les étapes de procédé consistant à chauffer et à filtrer, et établir une teneur en flavanes condensables entre 5 et 100 mg/ml dans la solution obtenue.

2. Utilisation de l'extrait selon la revendication 1 pour produire un médicament pour le traitement des maladies allergiques.

3. Utilisation de l'extrait selon la revendication 1 pour produire un médicament pour le traitement de l'asthme intrinsèque ou extrinsèque.
